(19) Europäisches Patentamt
European Patent Office
Office européen des brevets

(11) Veröffentlichungsnummer: **0 160 865**
**B1**

(12) # EUROPÄISCHE PATENTSCHRIFT

(45) Veröffentlichungstag der Patentschrift: **20.04.88**

(51) Int. Cl.⁴: **C 07 D 213/75**, C 07 C 59/105, A 61 K 31/44, A 61 K 31/19

(21) Anmeldenummer: **85104746.4**

(22) Anmeldetag: **19.04.85**

(54) **2-Amino-3-ethoxycarbonylamino-6-(p-fluor-benzylamino)-pyridingluconat und pharmazeutische Zubereitungen, die diese Substanz enthalten.**

(30) Priorität: **05.05.84 DE 3416609**

(43) Veröffentlichungstag der Anmeldung:
**13.11.85 Patentblatt 85/46**

(45) Bekanntmachung des Hinweises auf die Patenterteilung:
**20.04.88 Patentblatt 88/16**

(84) Benannte Vertragsstaaten:
**AT BE CH DE FR GB IT LI LU NL SE**

(56) Entgegenhaltungen:
**GB - A - 2 084 138**

**Die Akte enthält technische Angaben, die nach dem Eingang der Anmeldung eingereicht wurden und die nicht in dieser Patentschrift enthalten sind.**

(73) Patentinhaber: **Degussa Aktiengesellschaft, Weissfrauenstrasse 9, D-6000 Frankfurt am Main 1 (DE)**

(72) Erfinder: **Hettche, Helmut, Dr., Buchrainweg 65, D-6050 Offenbach (DE)**
Erfinder: **Emig, Peter, Dr., Taunusstrasse 6, D-6369 Niederdorfelden (DE)**
Erfinder: **Engel, Jürgen, Dr., Cranachstrasse 25, D-8755 Alzenau (DE)**

EP 0 160 865 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Das 2-Amino-3-ethoxycarbonylamino-6-(p-fluor-benzyl-amino)-pyridin-hydrochlorid ist in der Deutschen Patentschrift 1795858 beschrieben. Die Verbindung ist antiphlogistisch und analgetisch wirksam. Zur Herstellung pharmazeutischer Zubereitungen ist das in der Deutschen Offenlegungsschrift P 3133519.5 beschriebene 2-Amino-3-ethoxycarbonylamino-6-(p-fluor-benzylamino)-pyridin-maleat geeignet.

Um den Wirkstoff in Form einer Lösung zu applizieren, sind diese Salze aufgrund ihrer geringen Löslichkeit, schlechten Stabilität in Lösung und ungenügenden venösen Verträglichkeit jedoch wenig geeignet.

Es ist daher Aufgabe der Erfindung, weitere geeignete Zubereitungsformen für das 2-Amino-3-ethoxycarbonylamino-6-(p-fluor-benzylamino)-pyridin, beispielsweise injizierbare Lösungen bereitzustellen.

Die Erfindung betrifft die durch die Ansprüche definierten Gegenstände.

Es wurde überraschend gefunden, dass sich aus dem 2-Amino-3-ethoxycarbonylamino-6-(p-fluor-benzylamino)-pyridin-gluconat stabile pharmazeutische Zubereitungen, insbesondere Lösungen herstellen lassen. Es hat sich ausserdem gezeigt, dass das 2-Amino-3-ethoxycarbonylamino-6-(p-fluor-benzylamino)-pyridin-gluconat bei intravenöser Gabe eine bessere Verträglichkeit aufweist als die anderen bekannten Salze des 2-Amino-3-ethoxycarbonylamino-6-(p-fluor-benzylamino)-pyridins.

Das zur Herstellung des Gluconats verwendete 2-Amino-3-ethoxycarbonylamino-6-(p-fluor-benzylamino)-pyridin ist in der Deutschen Offenlegungsschrift P 3135519.5 beschrieben.

Die Herstellung des 2-Amino-3-ethoxycarbonyl-amino-6-(p-fluor-benzylamino)-pyridin-gluconats erfolgt durch Umsetzung von 2-Amino-3-ethoxy-carbonylamino-6-(p-fluor-benzylamino)-pyridin mit Gluconsäure oder Gluconsäure-δ-lacton. Diese Umsetzung kann mit oder ohne Lösungsmittel bei Temperaturen zwischen 20 und 140 °C, vorzugsweise 50 und 120 °C erfolgen.

Als Lösungsmittel kommen beispielsweise niedere Alkohole (zum Beispiel mit 1–6 C-Atomen wie Methanol, Ethanol, n-Propanol, Isopropanol, n-Butanol, 1,2-Propandiol, 1,4-Butandiol, n-Pentanol, 3-Pentanol, n-Hexanol), niedere Ketone (zum Beispiel mit 1–6 C-Atomen wie Aceton, 2-Butanon, Methylisobutylketon), Polyalkylenglykole (wobei Alkylen 2,3 oder 4 C-Atome bedeutet) mit Molekulargewichten zwischen 190 und 7500 und cyclische gesättigte Alkohole mit 4 bis 8, vorzugsweise 4 bis 6 C-Atomen wie zum Beispiel Glycofurol sowie pharmazeutisch verwendbare organische Säuren und Mischungen dieser Mittel mit Wasser in Frage.

Falls man anstelle von Gluconsäure das Gluconsäure-δ-lacton einsetzt, muss eine gewisse Menge Wasser vorhanden sein (zum Beispiel 1 Mol Wasser pro 1 Mol Lacton). Es ist beispielsweise vorteilhaft, das Gluconsäure-δ-lacton einzusetzen, da es handelsüblich in kristalliner Form vorliegt und schnell in Wasser zur Gluconsäure hydrolysiert wird. Beispielsweise kann die Herstellung auf folgende Weise erfolgen:

1 Mol 2-Amino-3-ethoxycarbonylamino-6-(p-fluor-benzylamino)-pyridin wird gegebenenfalls in einem Lösungsmittel (Ethanol, 1,2-Propandiol) suspendiert und mit einer bei einer Temperatur zwischen 20 und 140 °C bereiteten Lösung von 1 Mol Gluconsäure-δ-lacton in einem wasserhaltigen Lösungsmittel (Ethanol, 1,2-Propandiol) oder mit 1 Mol Gluconsäure mit oder ohne Lösungsmittel versetzt. Das Reaktionsgemisch lässt man noch einige Zeit, vorzugsweise unter Erwärmen (50 bis 70 °C), nachreagieren und verdampft dann gegebenenfalls vorhandene Lösungsmittel. Der verbleibende Rückstand wird mit Alkohol nachgedampft und bei erhöhter Temperatur im Vakuum getrocknet.

Das Salz wird in einer Ausbeute von 90% gewonnen. F: 117 bis 123 °C.

Das 2-Amino-3-ethoxycarbonylamino-6-(p-fluor-benzylamino)-pyridin-gluconat ist stabil und zeigt keine Neigung zur Verfärbung durch Reaktion mit der freien Base bzw. durch Oxidation, wie es beispielsweise bei dem Hydrochlorid zu beobachten ist.

Das 2-Amino-3-ethoxycarbonylamino-6-(p-fluor-benzylamino)-pyridin-gluconat ist daher besonders gut zur Herstellung pharmazeutischer Zubereitungen geeignet. Das 2-Amino-3-ethoxycarbonylamino-6-(p-fluor-benzylamino)-pyridin-gluconat ist schwer löslich in Wasser, durch den Zusatz einer physiologisch verträglichen organischen Säure, beispielsweise von Milchsäure, Glucuronsäure und/oder Gluconsäure wird die Löslichkeit gesteigert. Insbesondere durch den Zusatz von überschüssiger Gluconsäure wird die Löslichkeit erheblich gesteigert.

Zur Herstellung von stabilen, injizierbaren Lösungen des 2-Amino-3-ethoxycarbonylamino-6-(p-fluor-benzylamino)-pyridin-gluconats eignen sich als Lösungsmittel physiologisch verträgliche Mittel zum Beispiel Polyethylenglykol-Wasser-Mischungen oder Glycofurol-Wasser-Mischungen und Polyethylenglykol-Glycofurol-Wasser-Mischungen mit gegebenenfalls zusätzlichen üblichen Stabilisierungsmittel und Hilfsstoffen. Vorzugsweise enthalten solche Lösungen überschüssige freie Gluconsäure.

Beispielsweise wählt man einen mindestens 1-fachen, insbesondere 1- bis 6-fachen, vorzugsweise 2- bis 4-fachen molaren Überschuss (bezogen auf das 2-Amino-3-ethoxycarbonylamino-6-(p-fluor-benzylamino)-pyridin-gluconat) von Gluconsäure.

Die Gluconsäure kann beispielsweise durch Erhitzen einer wässrigen Lösung von Gluconsäure-δ-lacton auf 60 bis 70 °C über mindestens 30 min hergestellt werden.

Das Gluconsäure-δ-lacton liegt in kristalliner Form vor, so dass es im allgemeinen für die Praxis günstiger ist, die Gluconsäure jeweils durch Hydrolyse des Lactons herzustellen. Durch die Säurezugabe wird ein saurer pH-Wert bewirkt. Es

empfiehlt sich hierbei einen pH-Wert von 2,0 nicht zu unterschreiten, da sich sonst die für eine venöse Applikation erforderliche Verträglichkeit verschlechtert.

Lösungen des 2-Amino-3-ethoxycarbonylamino-6-(p-fluor-benzylamino)-pyridin-gluconats in den angegebenen Lösungsmitteln können auch dadurch erhalten werden, dass man das 2-Amino-3-ethoxycarbonylamino-6-(p-fluor-benzylamino)-pyridin zusammen mit überschüssiger Gluconsäure oder Gluconsäure-δ-lacton und dem betreffenden Lösungsmittel (Polyethylenglykol-Wasser, Glycofurol-Wasser, Polyethylenglykol-Glycofurol-Wasser) vermischt (z. B. durch Rühren oder Suspendieren bei einer Temperatur zwischen 20 und 80 °C unter Stickstoffbegasung).

Falls hierbei anstelle von Gluconsäure das Gluconsäure-δ-lacton eingesetzt wird, ist die Anwesenheit von Wasser erforderlich, und zwar mindestens 1 Mol Wasser, bezogen auf 1 Mol Gluconsäure-δ-lacton.

Die erfindungsgemässen 2-Amino-3-ethoxycarbonylamino-6-(p-fluor-benzylamino)-pyridin-gluconat-Lösungen enthalten z. B. 10 bis 50, insbesondere 20 bis 40, vorzugsweise 35 mg der freien Base pro ml. Beispielsweise ist der Gehalt an 2-Amino-3-ethoxycarbonylamino-6-(p-fluor-benzylamino)-pyridin-gluconat in einer injizierbaren Lösung etwa 55 mg/ml.

Die Lösungen mit einem 3-fachen bzw. 4-fachen molaren Überschuss an Gluconsäure zeigen einen pH-Wert von 3 bis 4 und verbleiben auch bei 7-tägiger Aufbewahrungszeit bei 25 °C und Tageslicht sowie nach 7-tägigem Stehen im Kühlschrank bei 7 °C ohne Kristallbildung bzw. Trübung klar gelöst. Die erfindungsgemäss hergestellten Lösungen weisen demnach Vorteile in der Stabilität und in der verbesserten Lagerhaltung auf.

Als Lösungsmittel kommen z. B. in Frage Polyethylenglykol-Wasser-Mischungen, die 10 bis 95%, insbesondere 30 bis 60, vorzugsweise 40% Polyethylenglykol (stets Gewichtsprozent) enthalten (das verwendete Polyethylenglykol hat vorzugsweise ein durchschnittliches Molekulargewicht von 200 bis 600, insbesondere 300 bis 400) oder Glycolfurol-Wasser-Mischungen, die 10 bis 95%, insbesondere 40% Glycofurol enthalten. Es kommen auch Polyethylenglykol-Glycofurol-Wasser-Mischungen in Frage. Eine besonders bevorzugte Ausführungsform enthält etwa 40% Polyethylenglykol mit einem durchschnittlichen Molekulargewicht von etwa 400 und 60% Wasser oder 20% Glycofurol und 80% Wasser.

Die erfindungsgemäss verwendeten Polyethylenglykole haben die allgemeine Formel

$$H(OCH_2CH_2)_nOH$$

und sind beispielsweise als Carbowax oder Lutrol im Handel erhältlich (siehe H. P. Fiedler Editio Cantor Aulendorf, 1981 Lexikon der Hilfsstoffe für Pharmazie, Kosmetik und angrenzende Gebiete 1981, Seite 726ff).

Die zur Anwendung kommenden Polyethylenglykole können z. B. folgende Molgewichtsbereiche haben:

Tabelle I

| Polyethylenglykole | Molekulargewichtsbereiche |
|---|---|
| 200 | 190–210 |
| 300 | 285–315 |
| 400 | 380–420 |
| 600 | 570–630 |
| 1000 | 950–1050 |
| 1540 | 1300–1600 |
| 4000 | 3000–4800 |
| 6000 | 5600–7500 |

Glycofurol-(Tetrahydrofurfurylalkohol-polyethylenglykolether) besitzt ein Molekulargewicht von ca. 190, und hat eine niedrige Toxizität und ist deshalb als Medium für Injektionslösungen geeignet [R. Budden Arzneimittel-Forschung 28 (II), 1586 (1978)]. Dieses Lösungsmittel ist ebenfalls im Handel erhältlich.

Die erfindungsgemässen Injektionslösungen können daneben noch Stabilisatoren und/oder Antioxidantien wie z. B. Natriumdisulfit, Aceton-Natriumdisulfit, Ethylendiamintetraessigsäure, Ascorbinsäure und ähnliche, enthalten.

Gemäss einer bevorzugten erfindungsgemässen Ausführungsform sind die Lösungen steril und in sterilen Behältern, beispielsweise in Ampullen, abgefüllt. Mittel zum Sterilisieren dieser Lösungen sind nach dem Stand der Technik bekannt. Es wird bevorzugt, die Lösungen unter aseptischen Bedingungen sowie unter steriler Stickstoffbegasung in Ampullen abzufüllen.

Synthese von 2-Amino-3-ethoxycarbonylamino-6-(p-fluor-benzylamino)-pyridin-gluconat.

Beispiel 1

3,043 g (0,01 Mol) 2-Amino-3-ethoxycarbonylamino-6-(p-fluorbenzylamino)-pyridin werden in 10 ml absol. Ethanol unter Stickstoffbegasung suspendiert und mit einer bei 60 °C im Verlaufe von 30 min bereiteten Lösung von 1,781 g (0,01 Mol Gluconsäure-δ-lacton in 10 ml Wasser) versetzt. Man erhitzt ca. 15 min auf 70 °C, bis eine klare Lösung entstanden ist und engt sodann im Vakuum bei 60 °C zur Trockne ein. Der verbleibende Rückstand wird mit 10 ml absol. Ethanol nachgedampft und im Vakuum bei 50 °C getrocknet.

Ausbeute 4,33 g (90% der Theorie), F: 117–123 °C, Löslichkeit in Wasser ca. 0,01%.

IR-Spektrum in KBr: siehe Fig. 1 (I, II)

Beispiel 1a

«Schmelzreaktion»

3,043 g (0,01 Mol) 2-Amino-3-ethoxycarbonylamino-6-(p-fluorbenzylamino)-pyridin werden mit 1,96 g (0,01 Mol) Gluconsäure intensiv vermischt und unter Stickstoffatmosphäre 30 min auf 140 °C erhitzt. Man lässt die Schmelze abkühlen, reibt mit wenig 50%igem Ethanol an und filtriert. Es wird

mit wenig kaltem Ethanol gewaschen und im Vakuum bei 50 °C getrocknet.

Ausbeute 4,1 g, F: 116–123 °C.

Eine Ampulle enthält beispielsweise 164,5 mg 2-Amino-3-ethoxycarbonylamino-6-(p-fluorbenzylamino)-pyridin-gluconat.

Beispiel 2

Der Herstellungsgang gilt für einen Ansatz zu 20 l (= 6500 Ampullen):

Herstellungsgang:

1. 10,0 l Wasser werden auf 70 °C erwärmt und nach Zugabe von 1562,0 g Gluconsäure-delta-Lacton wird die Lösung eine Stunde bei 70 °C belassen. Die Lösung wird dabei mit Stickstoff begast.
2. In Lösung 1 werden 8000,0 g Polyethylenglykol Molekulargewicht 380 bis 420 eingewogen und die Lösung unter Stickstoffbegasung auf 70 °C erwärmt.
3. 30,0 g Natriumdisulfit werden in 500,0 ml mit Stickstoff begastem Wasser gelöst.
4. Lösung 3 wird zu Lösung 2 gegeben.
5. 666,6 g 2-Amino-3-ethoxycarbonylamino-6-(p-fluorbenzylamino)-pyridin werden durch ein Sieb mit der Maschenweite 0,3 mm gesiebt und in Lösung 4 unter intensiver Stickstoffbegasung gelöst.
6. Lösung 5 wird abgekühlt und ad 20 l mit Stickstoff begastem Wasser aufgefüllt.
7. Lösung 6 wird durch ein Membranfilter der Porenweite 0,2 µm mit Glasfaservorfilter steril filtriert.
8. Inprozesskontrolle: Messung des Sauerstoffgehaltes von Lösung 7 mittels Sauerstoffelektrode. Messung des pH-Wertes von Lösung 7.
9. Lösung 7 wird unter aseptischen Bedingungen sowie unter Stickstoffbegasung in farblose Ampullen, Inhalt 3 ml, abgefüllt.

Eine Ampulle enthält 164,5 mg 2-Amino-3-ethoxycarbonylamino-6-(p-fluorbenzylamino)-pyridin-gluconat in 3 ml Lösung.

**Patentansprüche**

1. 2-Amino-3-ethoxycarbonylamino-6-(p-fluorbenzylamino)-pyridin-gluconat mit der chemischen Formel

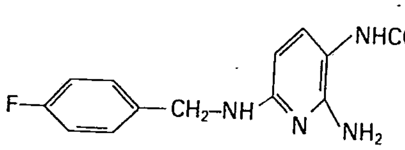

.$C_6H_{12}O_7$

2. Verfahren zur Herstellung von 2-Amino-3-ethoxycarbonylamino-6-(p-fluorbenzylamino)-pyridin-gluconat, mit der Formel I dadurch gekennzeichnet, dass man 2-Amino-3-ethoxycarbonylamino-6-(p-fluorbenzylamino)-pyridin mit oder ohne Lösungsmittel mit Gluconsäure oder mit Gluconsäure-δ-lacton in einem Lösungsmittel/Wasser-Gemisch bei Temperaturen zwischen 20 bis 140 °C umsetzt.

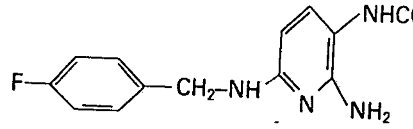

.$C_6H_{12}O_7$

3. Arzneimittel dadurch gekennzeichnet, dass es als Wirkstoff 2-Amino-3-ethoxycarbonylamino-6-(p-fluorbenzylamino)-pyridin-gluconat zusammen mit einem üblichen pharmazeutischen Träger und/oder einem Verdünnungsmittel enthält.

4. Arzneimittel nach einem oder mehreren der vorangegangenen Ansprüche, dadurch gekennzeichnet, dass das Arzneimittel eine injizierbare Lösung ist.

5. Injizierbare Lösungen des 2-Amino-3-ethoxycarbonylamino-6-(p-fluorbenzylamino)-pyridingluconats nach einem oder mehreren der vorangegangenen Ansprüche, dadurch gekennzeichnet, dass das Lösungsmittel aus einer Mischung von 10% bis 95% Polyethylenglykol und 5% bis etwa 90% Wasser besteht.

6. Injizierbare Lösungen des 2-Amino-3-ethoxycarbonylamino-6-(p-fluorbenzylamino)-pyridingluconats nach einem oder mehreren der vorangegangenen Ansprüche, dadurch gekennzeichnet, dass das Lösungsmittel aus einer Mischung von 10% bis 95% Glycofurol und 5% bis etwa 90% Wasser besteht.

7. Injizierbare Lösungen des 2-Amino-3-ethoxycarbonylamino-6-(p-fluorbenzylamino)-pyridingluconats nach einem oder mehreren der vorangegangenen Ansprüche, dadurch gekennzeichnet, dass das Lösungsmittel aus einer Mischung von 10% bis 90% Polyethylenglykol und 5% bis 85% Glycofurol und 5% bis etwa 85% Wasser besteht.

8. Injizierbare Lösungen des 2-Amino-3-ethoxycarbonylamino-6-(p-fluorbenzylamino)-pyridingluconats nach einem oder mehreren der vorangegangenen Ansprüche, dadurch gekennzeichnet, dass sie eine pharmazeutisch verträgliche organische Säure enthalten.

9. Injizierbare Lösungen des 2-Amino-3-ethoxycarbonylamino-6-(p-fluorbenzylamino)-pyridingluconats nach einem oder mehreren der vorangegangenen Ansprüche, dadurch gekennzeichnet, dass sie überschüssige freie Gluconsäure enthalten.

10. Verfahren zur Herstellung eines Arzneimittels, enthaltend das 2-Amino-3-ethoxycarbonylamino-6-(p-fluorbenzylamino)-pyridin-gluconat, dadurch gekennzeichnet, dass man das 2-Amino-3-ethoxycarbonylamino-6-(p-fluorbenzylamino)-pyridin-gluconat mit üblichen pharmazeutischen Hilfsstoffen und Trägern oder Verdünnungsmitteln vermischt.

11. Verfahren zur Herstellung eines Arzneimittels enthaltend das 2-Amino-3-ethoxycarbonylamino-6-(p-fluorbenzylamino)-pyridin-gluconat, dadurch gekennzeichnet, dass man 2-Amino-3-ethoxycarbonylamino-6-(p-fluorbenzylamino)-pyridin oder 2-Amino-3-ethoxycarbonylamino-6-(p-fluorbenzylamino)-pyridin-gluconat zusammen mit überschüssiger Gluconsäure oder mit Gluconsäure-δ-lacton in einem physiologisch verträglichen Lösungsmittel vermischt.

**Claims**

1. 2-Amino-3-ethoxycarbonylamino-6-(p-fluorobenzylamino)-pyridine gluconate corresponding to the following formula

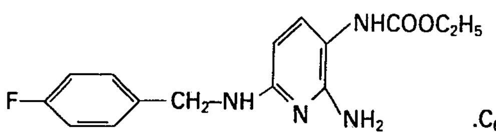

$.C_6H_{12}O_7$

2. A process for the production of 2-amino-3-ethoxycarbonylamino-6-(p-fluorobenzylamino)-pyridine gluconate corresponding to the formula I, characterized in that 2-amino-3-ethoxycarbonylamino-6-(p-fluorobenzylamino)-pyridine is reacted with gluconic acid in the presence or absence of a solvent or with gluconic acid δ-lactone in a solvent/water mixture at temperatures of 20 to 140 °C.

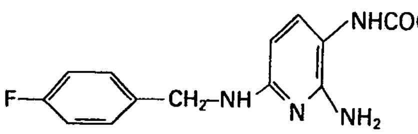

$.C_6H_{12}O_7$

3. A medicament, characterized in that it contains as active principle 2-amino-3-ethoxycarbonylamino-6-(p-fluorobenzylamino)-pyridine gluconate together with standard pharmaceutical excipients and/or diluents.

4. A medicament as claimed in one or more of the preceding claims, characterized in that the medicament is an injectable solution.

5. Injectable solutions of 2-amino-3-ethoxycarbonylamino-6-(p-fluorobenzylamino)-pyridine gluconate as claimed in one or more of the preceding claims, characterized in that the solvent consists of a mixture of 10 to 95% polyethylene glycol and 5 to about 90% water.

6. Injectable solutions of 2-amino-3-ethoxycarbonylamino-6-(p-fluorobenzylamino)-pyridine gluconate as claimed in one or more of the preceding claims, characterized in that the solvent consists of a mixture of 10 to 95% glycofurol and 5 to about 90% water.

7. Injectable solutions of 2-amino-3-ethoxycarbonylamino-6-(p-fluorobenzylamino)-pyridine gluconate as claimed in one or more of the preceding claims, characterized in that the solvent consists of a mixture of 10 to 90% polyethylene glycol, 5 to 85% glycofurol and 5 to about 85% water.

8. Injectable solutions of 2-amino-3-ethoxycarbonylamino-6-(p-fluorobenzylamino)-pyridine gluconate as claimed in one or more of the preceding claims, characterized in that they contain a pharmaceutically acceptable organic acid.

9. Injectable solutions of 2-amino-3-ethoxycarbonylamino-6-(p-fluorobenzylamino)-pyridine gluconate as claimed in one or more of the preceding claims, characterized in that they contain excess free gluconid acid.

10. A process for the production of a medicament containing 2-amino-3-ethoxycarbonylamino-6-(p-fluorobenzylamino)-pyridine gluconate, characterized in that 2-amino-3-ethoxycarbonylamino-6-(p-fluorobenzylamino)-pyridine gluconate is mixed with standard pharmaceutical auxiliaries and excipients or diluents.

11. A process for the production of a medicament containing 2-amino-3-ethoxycarbonylamino-6-(p-fluorobenzylamino)-pyridine gluconate, characterized in that 2-amino-3-ethoxycarbonylamino-6-(p-fluorobenzylamino)-pyridine or 2-amino-3-ethoxycarbonylamino-6-(p-fluorobenzylamino)-pyridine gluconate is mixed together with excess gluconic acid or with gluconic acid δ-lactone in a physiologically acceptable solvent.

**Revendications**

1. Gluconate de 2-amino-3-éthoxycarbonylamino-6-(p-fluoro-benzylamino)-pyridine de formule chimique

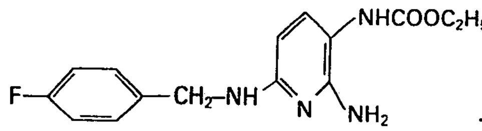

$.C_6H_{12}O_7$

2. Procédé pour la préparation de gluconate de 2-amino-3-éthoxycarbonylamino-6-(p-fluoro-benzylamino)-pyridine de formule I, caractérisé en ce qu'on fait réagir la 2-amino-3-éthoxycarbonylamino-6-(p-fluoro-benzylamino)-pyridine, avec ou sans solvant, avec l'acide gluconique ou avec la δ-gluconolactone dans un mélange solvant/eau à une température comprise entre 20 et 140 °C.

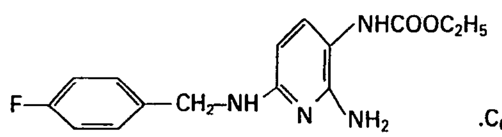

$.C_6H_{12}O_7$

3. Médicament, caractérisé en ce qu'il contient, en tant que substance active, du gluconate de 2-amino-3-éthoxycarbonylamino-6-(p-fluoro-benzylamino)-pyridine en combinaison avec un excipient pharmaceutique et/ou un diluant usuel.

4. Médicament selon une ou plusieurs des revendications précédentes, caractérisé en ce que ce médicament est une solution injectable.

5. Solutions injectables du gluconate de 2-amino-3-éthoxycarbonylamino-6-(p-fluoro-benzylamino)-pyridine selon une ou plusieurs des revendications précédentes, caractérisées en ce que le solvant est constitué par un mélange de 10% à 95% de polyéthylèneglycol et de 5% à environ 90% d'eau.

6. Solutions injectables du gluconate de 2-amino-3-éthoxycarbonylamino-6-(p-fluoro-benzylamino)-pyridine selon une ou plusieurs des revendictations précédentes, caractérisées en ce que le solvant est constitué par un mélange de 10% à 95% de Glycofurol et de 5% à environ 90% d'eau.

7. Solutions injectables de gluconate de 2-amino-3-éthoxycarbonylamino-6-(p-fluoro-benzylamino)-pyridine selon une ou plusieurs des revendications précédentes, caractérisées en ce que le solvant est constitué par un mélange de 10% à

90% de polyéthylèneglycol, de 5% à 85% de Glycofurol et de 5% à environ 85% d'eau.

8. Solutions injectables de gluconate de 2-amino-3-éthoxycarbonylamino-6-(p-fluoro-benzylamino)-pyridine selon une ou plusieurs des revendications précédentes, caractérisées en ce qu'elles contiennent un acide organique acceptable pharmaceutiquement.

9. Solutions injectables du gluconate de 2-amino-3-éthoxycarbonylamino-6-(p-fluoro-benzylamino)-pyridine selon une ou plusieurs des revendications précédentes, caractérisées en ce qu'elles contiennent de l'acide gluconique libre en excès.

10. Procédé pour la préparation d'un médicament contenant du gluconate de 2-amino-3-éthoxycarbonylamino-6-(p-fluoro-benzylamino)-pyridine, caractérisé en ce qu'on mélange le gluconate de 2-amino-3-éthoxycarbonylamino-6-(p-fluoro-benzylamino)-pyridine avec des adjuvants et excipients ou diluants pharmaceutiques usuels.

11. Procédé pour la préparation d'un médicament contenant du gluconate de 2-amino-3-éthoxycarbonylamino-6-(p-fluoro-benzylamino)-pyridine, caractérisé en ce qu'on mélange de la 2-amino-3-éthoxycarbonylamino-6-(p-fluoro-benzylamino)-pyridine ou du gluconate de 2-amino-3-éthoxycarbonylamino-6-(p-fluoro-benzylamino)-pyridine avec de l'acide gluconique en excès ou avec de la δ-gluconolactone dans un solvant acceptable physiologiquement.

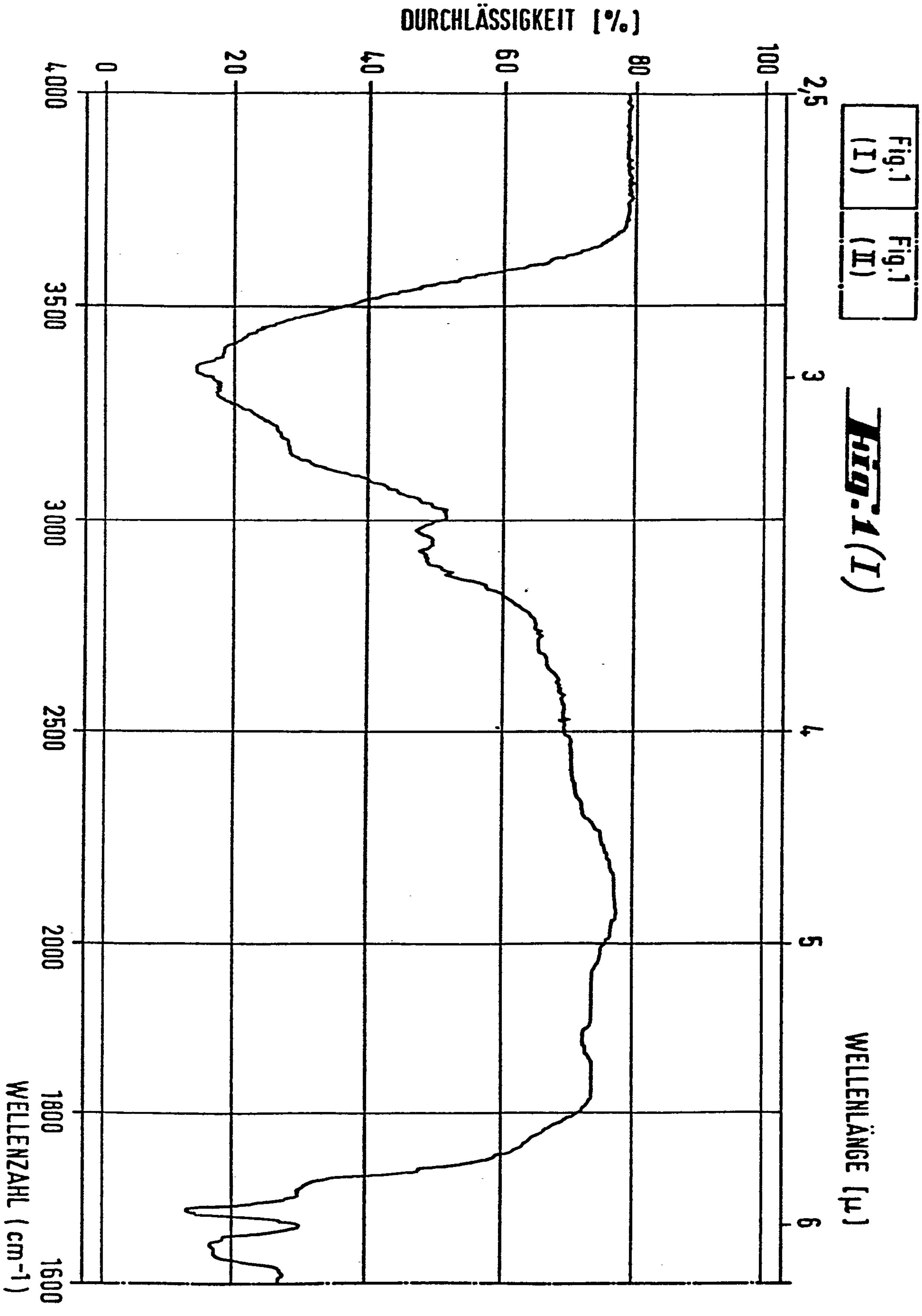
DURCHLÄSSIGKEIT [%]
0
20
40
60
80
100
4000
3500
3000
2500
2000
1800
1600
WELLENZAHL (cm-1)
2,5
3
4
5
6
WELLENLÄNGE [µ]
Fig.1 (I)
Fig.1 (II)

Fig.1(I)

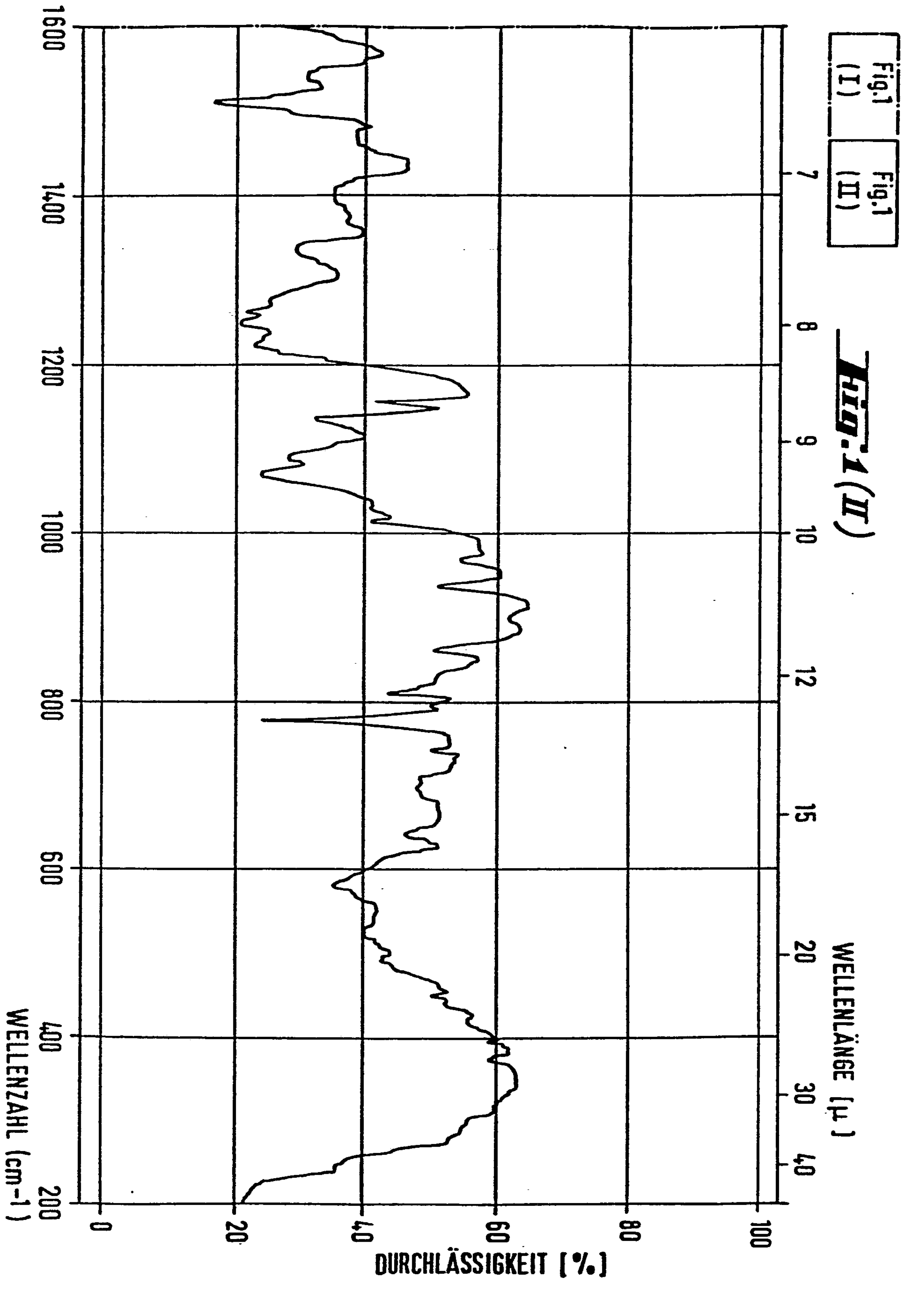

Fig.1 (I)
Fig.1 (II)
Fig.1 (II)
1600
1400
1200
1000
800
600
400
200
WELLENZAHL (cm-1)
7
8
9
10
12
15
20
30
40
WELLENLÄNGE [μ]
0
20
40
60
80
100
DURCHLÄSSIGKEIT [%]